**Europäisches Patentamt**

**European Patent Office**

**Office européen des brevets**

(19)

(11) Numéro de publication: **0 085 016 B1**

# FASCICULE DE BREVET EUROPEEN

(12)

(45) Date de publication du fascicule du brevet:
28.10.87

(51) Int. Cl.⁴: **A 61 M 1/34**

(21) Numéro de dépôt: **83420002.4**

(22) Date de dépôt: **07.01.83**

(54) **Appareillage utilisable en plasmaphérèse avec appareil à membrane semi-perméable.**

(30) Priorité: **11.01.82 FR 8200485**

(43) Date de publication de la demande:
**03.08.83 Bulletin 83/31**

(45) Mention de la délivrance du brevet:
**28.10.87 Bulletin 87/44**

(84) Etats contractants désignés:
**AT BE CH DE GB IT LI LU NL SE**

(56) Documents cités:
**WO - A - 79/01121**
**WO - A - 81/02979**
**DE - A - 2 745 041**
**DE - A - 2 939 213**
**DE - A - 3 043 682**
**DE - B - 2 100 209**
**FR - A - 2 198 759**
**US - A - 4 191 182**

(73) Titulaire: **RHONE-POULENC S.A., 25, quai Paul Doumer,
F-92408 Courbevoie (FR)**

(72) Inventeur: **Angleraud, René, 35, rue des Géraniums,
F-69320 Feyzin (FR)**
Inventeur: **Bouveret, Elisabeth, 21, rue Rousselet,
F-75007 Paris (FR)**
Inventeur: **Malbrancq, Jean-Michel, 7, rue Jeanne d'Arc,
F-94320 Thiais (FR)**

(74) Mandataire: **Vogt, Bernard et al, RHONE-POULENC
INTERSERVICES Service Brevets Chimie Centre de
Recherches de Saint-Fons B.P. 62, F-69192 Saint-Fons
Cédex (FR)**

## Description

La présente invention concerne un appareillage utilisable notamment en plasmaphérèse et comprenant un appareil séparateur à membrane.

La plasmaphérèse est une opération consistant à séparer le sang entier d'un donneur en deux parties, la première partie constituant la phase plasmatique, tandis que la seconde partie constitue la phase cellulaire qui est en général réinjectée au donneur. La phase plasmatique est une solution aqueuse complexe contenant notamment des protéines, tandis que la phase cellulaire contenant encore du plasma comprend les globules rouges (ou hématies), les globules blancs (ou leucocytes) et les plaquettes.

La technique de plasmaphérèse est utilisée depuis fort longtemps en expérimentation animale. A titre indicatif on peut citer l'article de John J. ABEL et al. ayant pour titre «Plasma removal with return of corpuscles», paru dans J. Pharmacol. Exp. Ther. en 1914, no. 5, pages 625 à 641 dans lequel du sang de chien est centrifugé pour faire la séparation. On peut citer également l'article de A. GEIGER paru en 1931 dans J. Phys. 71, pages 111–120 ayant pour titre «Method of Ultrafiltration in vivo» dans lequel il est décrit une opération de plasmaphérèse en continu sur chien, l'appareil de séparation utilisé étant un séparateur à membrane, cette dernière étant disposée en spirale et étant choisie de façon telle qu'il soit possible d'obtenir une solution plasmatique comprenant la totalité des protéines du sang traité, lorsque cela est désiré.

La plasmaphérèsse est également appliquée à l'homme depuis un certain nombre d'années comme l'indique l'article «La plasmaphérèse – Technique – Indications» de Fr. OBERLING et al. paru dans J. Med. Strasbourg 1968, mars, page 277–279. La plasmaphérèse tend ainsi maintenant à supplanter le don total du sang car cette technique présente l'avantage de permettre de prélever à l'homme de plus grandes quantités de plasma sans inconvénient. Du fait que l'on restitue au donneur les éléments figurés du sang, les séances de prélèvement peuvent être plus rapprochées dans le temps que pour le don du sang.

Ainsi la plasmaphérèse est une technique ancienne et les perfectionnements ultérieurs lui ayant été apportés portent soit sur des appareils à centrifugation, soit sur des appareils à membranes. Parmi les brevets de perfectionnement concernant des appareils à membranes, on peut citer le brevet allemand DE-A-2 100 209 de AMICON dans lequel il est décrit un récipient comprenant une membrane formant une spirale pour la circulation du sang entier prélevé d'un donneur et dans lequel on exerce une pression sur le sang contenu dans le récipient, soit au moyen d'un gaz, soit au moyen du piston d'une seringue soumis à l'action d'un ressort à lame. Par rapport à l'appareil de GEIGER précédemment décrit, cet appareil de par sa conception présente l'inconvénient de ne pas permettre d'opérer en continu sur le donneur. On peut citer également le brevet américain 4 191 182 de HEMOTHERAPY Inc., dans lequel il est décrit un appareillage à membrane et dans lequel le sang prélevé en continu au donneur est séparé en plasma et en une fraction cellulaire retournant en continu au donneur, cet appareillage ayant notamment pour caractéristique le fait de permettre à une partie de la fraction cellulaire de recirculer dans le compartiment amont de l'appareil à membrane et le fait de permettre à la fraction plasmatique de recirculer dans le compartiment aval du même appareil. On peut citer de plus la demande internationale déposée par FRIEDMAN et al. et publiée sous le n° wo 79/01 121, dans laquelle il est également question d'un appareillage permettant de retirer le sang du donneur et de lui réinjecter la fraction n'ayant pas traversé la membrane, de façon continue.

Cependant les appareillages décrits ci-avant et permettant la plasmaphérèse en continu ont notamment pour inconvénient d'exiger de piquer le donneur en deux endroits distincts, ce qui est assez désagréable pour celui-ci.

Du document DE-A-2 745 041 est connu un appareillage utilisant un appareil à centrifugation et permettant des opérations de plasmaphérèse sur un donneur en ne le piquant qu'à un seul endroit avec une aiguille.

Un but de la présente invention est donc un appareillage permettant de soumettre le sang en provenance du donneur à une première séparation lors de son passage sur la membrane, puis à une seconde séparation sur la même membrane lors de son retour au donneur.

Un autre but de la présente invention est un appareillage spécialement adapté permettant d'obtenir un plasma de très bonne qualité et dans les meilleures conditions de rendement de filtration, tout en assurant une hémolyse pratiquement nulle du sang.

Un autre but de la présente invention est un appareillage permettant de réguler la pression de sortie de la fraction cellulaire du compartiment amont de l'appareil à membrane à des valeurs comprises généralement entre 0 et 2 660 Pa (0 et 20 mm de mercure) en pression relative, le compartiment aval étant à la pression atmosphérique.

Un autre but de la présente invention est un appareillage permettant de retirer d'un donneur environ 600 ml de plasma en environ 45 minutes.

Un autre but de la présente invention est un appareillage de prélèvement de plasma dans lesquels il est possible d'adapter facilement la stratégie opératoire en fonction du donneur, des désirs de l'opérateur et des caractéristiques de l'appareil à membrane utilisée.

Il a maintenant été trouvé et c'est ce qui fait l'objet de la présente invention un appareillage utilisable notamment en plasmaphérèse, caractérisé en ce qu'il comprend:

– un dispositif 1 de prélèvement du sang d'un donneur,

–un appareil 2 à membrane séparant le sang en une fraction ayant traversé la membrane, et en une fraction n'ayant pas traversé la membrane,

– une première canalisation 5 reliant le dispositif 1 de prélèvement à l'entrée 6 du compartiment amont 3 de l'appareil 2 à membrane,

– une pompe 7 disposée sur cette canalisation 5,

– un capteur de pression 14 du sang circulant dans la canalisation 5, ledit capteur étant situé entre la pompe 7 et l'entrée 6 du compartiment amont 3,

– un garrot gonflable 21,

– des moyens permettant de gonfler ou de dégonfler le garrot

– une deuxième canalisation 16 reliant la sortie 15 du compartiment amont 3 de l'appareil 2 à membrane à un récipient 17 pour recueillir la fraction du sang n'ayant pas traversé la membrane,

– une pompe 18 disposée sur cette deuxième canalisation 16 et tournant dans le même sens que celui de la pompe 7,

– un capteur de pression 19 disposé sur la canalisation 16 entre ladite pompe 18 et la sortie 15 du compartiment amont 3 de l'appareil à membrane 2,

– un récipient 20 de recueil du plasma ayant traversé la membrane, ce récipient 20 étant en communication avec la sortie du compartiment aval 4 de l'appareil à membrane 2,

– des moyens pour connaître le volume de sang prélevé au donneur lors de la phase de prélèvement, au début de laquelle le garrot 21 est gonflé sur le membre du donneur,

– des moyens pour inverser le sens de rotation des pompes 7 et 18 lorsque la quantité souhaitée de sang est prélevée lors de la phase de prélèvement et assurer ainsi le retour du sang depuis le récipient 17 jusqu'au donneur en passant par les canalisations 16 et 5 et par le compartiment amont 3 de l'appareil à membrane 2, le garrot 21 étant dégonflé au début de la phase de retour,

– des moyens 27 permettant la vérification de la fin de la phase de retour et assurant la commutation d'une phase de retour à une phase de prélèvement.

La description de l'appareillage selon la présente invention sera mieux comprise à l'aide des figures ci-jointes, qui illustrent, de façon schématique, à titre d'exemples non limitatifs et sans échelle déterminée, des modes de réalisation particuliers dudit appareillage.

La figure 1 représente un mode de réalisation d'un appareillage selon la présente invention.

La figure 2 représente un mode de réalisation d'un appareillage dans lequel ont été notamment portées les connexions à un dispositif de contrôle et de commande.

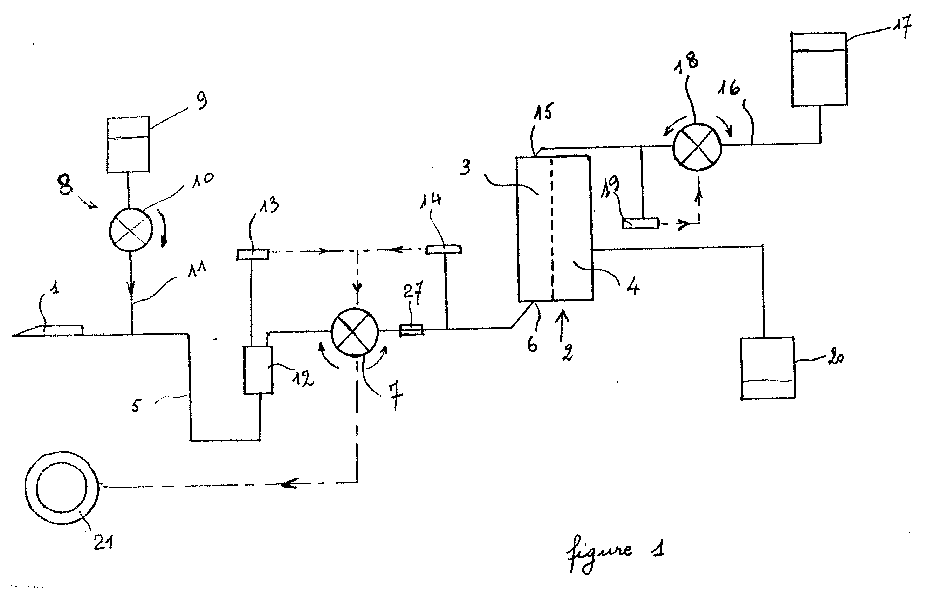

L'appareillage représenté figure 1 comprend un dispositif de prélèvement de sang au donneur, constitué d'une aiguille 1 de prise de sang. A titre indicatif cette aiguille peut avoir un diamètre interne prélèvement de sang au donneur, constitué d'une aiguille 1 de prise de sang. A titre indicatif cette aiguille peut avoir un diamètre extérieur de 1,65 mm et un diamètre interne de 1,45 mm, telle que celles répertoriées dans les centres de transfusion sanguine sous le vocable 16 G. Un appareil séparateur 2 à membrane, comprenant un compartiment amont 3 et un compartiment aval 4 est relié à l'aiguille 1 par une première canalisation 5 allant de l'aiguille 1 à l'entrée 6 du compartiment amont 3 de l'appareil à membrane. Cette canalisation 5 est généralement constituée par un tube en matière plastique, comme par exemple du polychlorure de vinyle. Sur cette canalisation ou ligne 5 se trouve une pompe 7 pouvant tourner dans les deux sens, avantageusement du genre pompe péristaltique. De telles pompes sont pas exemple commercialisées par la Société HOSPAL sous la dénomination RP 01 (marque commerciale déposée). Entre la pompe 7 et l'aiguille 1 est disposé un dispositif 8 destiné à transmettre un produit anticoagulant au sang en provenance du donneur, par exemple une solution glucosée contenant 35,6 g/l de citrate trisodique, référence AB 16 de la Société BIELUZ (marque commerciale déposée). Ce dispositif 8 comprend par exemple un réservoir 9 d'anticoagulant, une canalisation 11 raccordée à la canalisation 5 et au réservoir 9, et une pompe 10, par exemple du genre péristaltique, située sur la canalisation 11. Cette canalisation 11 se raccorde sur la canalisation 5 le plus près possible de l'aiguille 1. Entre le point de raccordement des canalisations 11 et 5 et la pompe 7, un détecteur 12 de bulles et un capteur 13 de pression sont sur la canalisation 5. Entre la pompe 7 et l'entrée 6 du compartiment amont 3 du séparateur 2 à membrane est également disposé un détecteur 14 de pression. La sortie 15 du compartiment amont 3 du séparateur 2 à membrane est reliée par une canalisation 16 à un récipient 17 de recueil de la partie du sang ayant passé au contact de la membrane sans la traverser. Cette canalisation 16 peut être en même matière et aux mêmes diamètres que la canalisation 5, tandis que le récipient 17 peut être constitué par une poche en matière plastique souple. Une pompe 18 est sur la canalisation ou ligne 16 et peut tourner dans les deux sens, cette pompe étant avantageusement de type péristaltique. Entre la sortie 15 du compartiment amont 3 du séparateur 2 à membrane et la pompe 18 se trouve un capteur de pression 19 sur la ligne 16. Le compartiment aval 4 du séparateur 2 est relié à un récipient 20 de recueil du plasma ayant traversé la membrane, ce récipient 20 étant par exemple une poche en matière plastique dont l'intérieur est en communication avec la pression atmosphérique, par exemple par un tampon stérile situé dans sa partie haute. Ce récipient 20 peut être également une poche en matière plastique souple, telle que celles vendues par la Société FENWAL sous la référence «transfer-pack» – R 2022 – (marque commerciale déposée).

L'appareil séparateur 2 dont il a été question ci-avant peut avoir sa membrane sous forme plane, sous forme spiralée, ou sous forme de petits tubes fins tels que des fibres creuses. Lorsque la membrane comprend une pluralité de fibres creuses, le sang circule avantageusement à l'intérieur des fibres creuses, l'ensemble des parties internes des fibres constituant le compartiment 3 amont du séparateur. Lorsque la membrane est sous forme plane ou spiralée, le sang circule avantageusement entre deux membranes ou des groupes de deux membranes qui constituent le compartiment amont 3 du séparateur 2.

Les membranes utilisées sont de préférence celles qui permettent de recueillir un plasma: – dans lequel toutes les protéines du sang initial sont retrouvées dans les mêmes proportions, – dont la concentration protéique est supérieure à 55,5 g/l, – dans lequel il n'y a pas de globules rouges et dans lequel la concentration plaquettaire est inférieure à 15 000 plaquettes par mm³. Les membranes choisies sont celles permettant également de ne pas hémolyser le sang circulant à leur contact, tout en offrant de bons rendements de filtration.

Ces membranes ont avantageusement un taux de rejet au latex, dont les particules sont calibrées à 0,27 microns, inférieur à 75% et elles ont un taux de rejet au latex, dont les particules sont calibrées à 0,64 microns, supérieur à 15%. Préférentiellement le taux de rejet aux particules calibrées à 0,27 microns est inférieur à 30% et le taux de rejet aux particules de latex calibrées à 0,64 microns est supérieur à 90%.

Pour effectuer cette mesure de taux de rejet au latex, on opère de la façon suivante, lorsque les membranes sont planes.

Dans une cellule de type AMICON Modèle 52 (marque commerciale déposée), on charge 50 ml de suspension de particules calibrées de polystyrène de diamètre 0,27–04 ou 0,64 microns (vendues par la Société RHONE-POULENC sous la marque commerciale déposée ESTAPOR) diluée à 0,1% de l'eau distillée additionnée de 1% d'agent tensio-actif (alkylarylsulfonate marque SINOZON NAS 60, fabriqué par la Société SINNOVA).

La cellule AMICON est équipée d'un échantillon de la membrane tramée. On établit une pression d'air correspondant à 20 cm d'eau. On récupère les six premiers millilitres de filtrat dont on détermine la concentration (cf) en particules calibrées.

Le taux de rejet est défini par la formule:

$$\frac{(0,1\text{-cf}) \times 100}{0,1}$$

Des membranes ayant les caractéristiques ci-avant sont généralement en matériau synthétique, par exemple en esters de cellulose (nitrate de cellulose...), en cellulose régénérée, en polycarbonate.... Ces membranes peuvent être également à base de polyétheruréthanes comprenant des groupements ammonium héparinés, ou en copolymère d'acrylonitrile. De façon avantageuse ces membranes sont renforcées par une trame lorsqu'elles sont sous forme de membranes planes et ont une épaisseur comprise entre 50 et 200 microns.

L'appareillage représenté figure 1 comprend de plus un garrot 21 gonflable de type connu en soi, ce garrot pouvant être gonflé lorsque cela est désiré, par un dispositif (également connu en soi) comprenant une électrovanne branchée sur une bouteille de gaz sous pression, par exemple de l'azote ou de fréon. Sur la figure 1 les lignes mixtes comprenant des flèches, entre les capteurs de pression 13, 14 et 19 et les pompes 7 et 18 signifient que lesdits capteurs agissent sur les pompes en fonction des consignes qui leur ont été données. Ainsi le fonctionnement des pompes est asservi aux consignes des capteurs. Il en est de même pour le garrot 21 dont le gonflage (ou le dégonflage) est asservi au sens de rotation de la pompe 7.

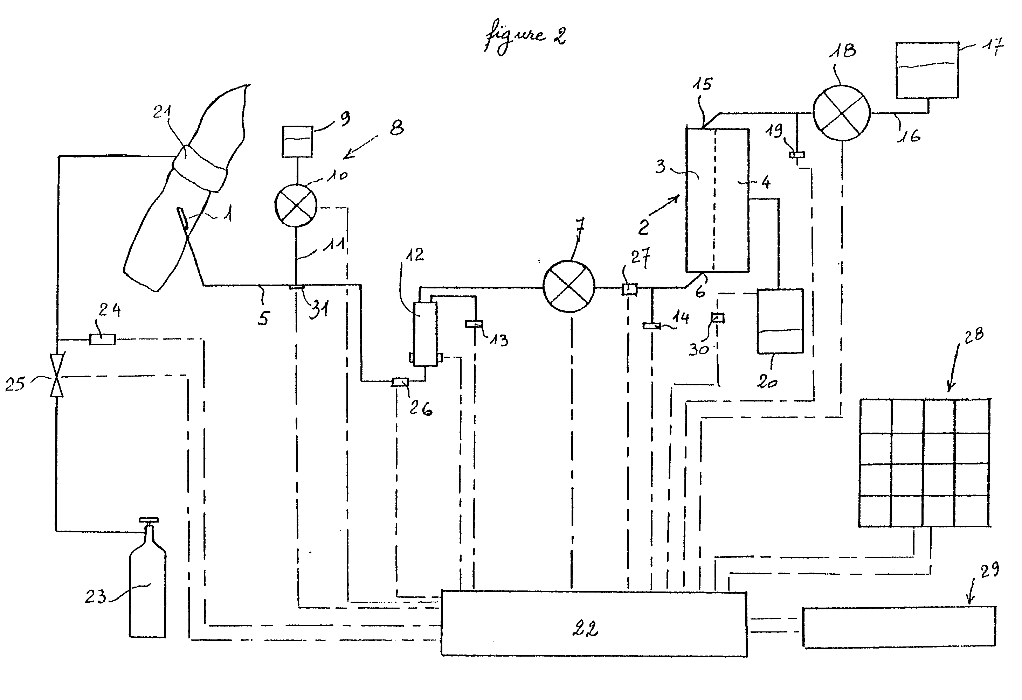

La figure 2 est une représentation d'un appareillage équivalent à celui de la figure 1, mais dans lequel on trouve les connexions électriques des différents éléments à une unité logique 22 de commande et de contrôle, les lignes électriques étant représentées en traits mixtes, l'unité 22 étant reliée à une source de courant (non représentée).

Pour la mise en œuvre de l'appareillage précédemment décrit on opère de la façon suivante. On commence par remplir la canalisation 11 de solution citratée et comme la jonction de la canalisation 11 avec la canalisation 5 est en fait très proche de l'aiguille 1, on peut considérer que l'aiguille 1 est au moins en partie remplie de cette solution citratée. Le garrot ayant été préalablement gonflé à la pression souhaitée aux environs de 7980 Pa (soit 60 mm de mercure) grâce à la bouteille 23 et au manomètre 24 à contact par ouverture de l'électrovanne 25 [ces deux derniers éléments étant connectés à l'unité logique 22], on enfonce l'aiguille 1 dans une veine du donneur après avoir préparé classiquement l'endroit de piqure, situé entre le garrot et l'extrémité du membre. A ce moment la pompe 10 envoie du citrate dans la canalisation 5, tandis que les deux pompes 7 et 18 tournent dans le même sens et mènent le sang du donneur jusqu'au récipient 17 en la faisant passer par le compartiment 3 amont du séparateur 2 à membrane où une partie du plasma traverse la membrane pour aller dans le compartiment aval 4 relié à la poche 20. Le capteur 13 de pression asservit la pompe 7 de façon à ce que la pression mesurée à cet endroit de la ligne 5 reste toujours supérieure à une certaine valeur, généralement proche de 0 Pa (0 mm de mercure), appelée pression de seuil, pour s'assurer que la pompe 7 ne «tire» pas le sang directement de la veine du donneur. Si la pression dans cette partie de la ligne devient inférieure à la consigne fixée au capteur 13, automatiquement l'unité logique 22 agit et arrête ou ralentit momentanément la rotation de la pompe 7, tant que la pression voulue n'est pas revenue. Le capteur de pression 14 est réglé de façon à ce qu'une pression à l'entrée 6 du compartiment amont 3 supérieure à une valeur maximale, comprise par exemple entre 5320 et 13 300 Pa (40 et 100 mm de mercure) en valeur relative, de préférence entre 7980 et 11 970 Pa (60 et 90 mm de mercure), soit automatiquement détectée. Si cette valeur maximale souhaitée est dépassée, automatiquement l'unité logique 22 arrête la pompe 7. Le capteur 19 permet d'assurer à la sortie 15 du compartiment amont 3 du séparateur 2 à membrane une pression comprise entre 0 et 2660 Pa (0 et 20 mm de mercure) en valeur relative, tandis que le compartiment aval 4 est à la pression atmosphérique. Si la pression de sortie 15 du compartiment 3 amont dépasse la pression maximale souhaitée, automatiquement l'unité logique 22 de commande et de contrôle agit sur la pompe 18 pour en accélérer la rotation du moteur, c'est-à-dire en augmenter le débit.

La période pendant laquelle le sang sort de la veine du donneur est appelée phase de prélèvement. Celle-ci se termine par exemple en fonction du volume prédéterminé de sang que l'on veut prélever au donneur, ce volume étant bien entendu toujours inférieur au volume du récipient 17 de recueil du sang. De façon avantageuse un dispositif tachymétrique est branché sur la pompe 7 et lorsque le volume souhaité de sang est prélevé au donneur, l'unité logique 22 agit et arrête les pompes 7, 10 et 18. L'unité logique agit alors simultanément sur l'électrovanne 25 qui se positionne de façon telle que le garrot 21 se dégonfle et elle met les pompes 7 et 18 en rotation dans le sens inverse de celui de la phase précédente, dite de prélèvement. Alors commence la phase dite de retour du sang au donneur, au cours de laquelle le sang contenu dans la poche 17 passe à nouveau dans le compartiment amont 3 de l'appareil à membrane 2. Au cours de la phase de retour, pendant laquelle la pompe 11 de citrate est arrêtée, le capteur 19 sert de sécurité et est réglé pour que la pression relative ne dépasse pas une certaine valeur fixée à l'avance, par exemple comprise entre 5320 et 13 300 Pas (40 et 100 mm de mercure), de préférence entre 7980 et 11 970 Pa (60 et 90 mm de mercure), lors de l'entrée du sang dans le compartiment 3 du séparateur 2 à membrane; le sang pénètre alors dans le séparateur par la tubulure 15 repérée sur les figures 1 et 2. Si cette valeur prédéterminée de pression est dépassée, l'unité logique 22 agit sur la pompe 18 et l'arrête au moins momentanément. Le capteur 14 assure que le sang sortant du séparateur par la tubulure 6 est à une pression relative comprise entre 0 et 2660 Pa (0 et 20 mm de mercure), tandis que le compartiment aval 4 du séparateur est à la pression atmosphérique. Si la valeur fixée est dépassée l'unité logique 22 agit immédiatement pour accélérer la rotation de pompe 7, c'est-à-dire augmenter son débit. Au cours de la phase de retour le sang provenant du récipient ou poche 17 déjà privé d'une partie de son plasma, est à nouveau filtré par passage au contact de la membrane et appauvri à nouveau d'une partie de plasma allant dans le compartiment aval 4 du séparateur 2, puis dans la poche 20. Le sang passe ensuite dans le détecteur 12 de bulles. Si des bulles sont décelées par ce dernier 12, l'unité logique 22 arrête immédiatement les pompes 7 et 18 et éventuellement agit sur un «clamp» 26, ou dispositif d'obturation, qui ferme la canalisation 5. Au cours de la phase de retour le capteur 13 de pression sert de sécurité en ce sens qu'il est réglé de façon telle que la pression du sang ne dépasse pas une certaine valeur prédéterminée. Si cette valeur est dépassée, par exemple par obstruction de l'aiguille 1, l'unité logique 22 intervient immédiatement pour arrêter la pompe 7. Au cours de la phase de retour le sang peut éventuellement passer au travers d'un filtre classique prévu dans le détecteur à bulles 12 pour éviter que des particules indésirables puissent être renvoyées au donneur. Ce filtre peut par exemple s'effacer lors de la phase de prélèvement, tandis qu'il se rabat sur un siège, prévu dans le détecteur de bulles, lors de la phase de retour. La fin de la phase de retour est détectée, par exemple par un détecteur optique 27 prévu sur la ligne 5. Lorsqu'il ne passe plus de sang (privé d'une partie de son plasma) à l'endroit où se trouve le détecteur 27, l'unité logique 22 intervient pour arrêter la phase de retour et faire repasser l'appareillage sur une phase de prélèvement. Ainsi les pompes 7 et 18 sont arrêtées et mises en mouvement en tournant toutes les deux dans le même sens, mais en sens inverse à celui de la phase de retour, tandis que le garrot 21 est gonflé et la pompe 10 de citrate mise en route. Lorsqu'en fin d'une phase de retour on s'aperçoit que la poche 20 à plasma contient une quantité suffisante de plasma, on arrête complètement l'opération.

Généralement le débit de la pompe 10 est réglé de façon que, lors de la phase dite de prélèvement, on ait un volume de citrate pour 8 volumes de sang ou de préférence 1 volume de citrate pour 16 volumes de sang, le rapport étant choisi par l'utilisateur. Ce rapport de dilution est avantageusement obtenu en asservissant la vitesse de rotation de la pompe 10 à celle de la pompe 7.

Il apparaît clairement que l'appareillage selon la présente invention peut faire l'objet d'une automatisation très poussée. Ainsi comme cela est représenté plus particulièrement sur la figure 2, l'unité logique 22 de commande et de contrôle peut être reliée à un clavier 28 et à un afficheur 29. De même l'unité logique 22 peut être reliée à un tableau synoptique (non représenté) sur lequel la localisation de toute anomalie est signalée par un voyant lumineux à l'opérateur, en même temps par exemple qu'un signal sonore est mis en route. Sur le clavier 28 à touches on peut choisir le volume de sang que l'on veut faire circuler pendant la phase de prélèvement (300, 350, 400, 450 cm$^3$ de sang par exemple) en appuyant sur la touche correspondante. On peut également choisir le volume de plasma que l'on veut prélever durant la séance (400, 500 ou 600 cm$^3$ par exemple) en appuyant sur la touche correspondante. Ainsi un dispositif 30 est avantageusement prévu sur la poche 20 de plasma pour connaître instantanément le volume (ou le poids) de plasma prélevé en cours de séance, ce dispositif 30 connu en soi étant connecté à l'unité logique 22. Sur le clavier 28 peut également être prévue une touche pour l'amorçage automatique de la ligne 11 avant de piquer le donneur. En appuyant sur cette touche la pompe 10 se met en route et s'arrête automatiquement lorsque la solution de citrate est décelée par exemple à la jonction 31 des deux lignes 11 et 5. Sur le clavier 28 on peut également prévoir par exemple une touche pour connaître sur l'afficheur 29 le volume instantané de plasma dans la poche 20 à tout moment, une touche pour connaître sur l'afficheur 29 le débit du sang de la pompe 7, pour connaître le temps de séance en cours..... L'appareillage peut comprendre, en liaison avec l'unité logique 22 et les valeurs affichées au clavier 28 en ce qui concerne le volume de sang souhaité pendant la phase de prélèvement et le volume total de plasma désiré, un système intégrateur agissant au cours de la dernière phase de prélèvement pour que le volume de prélèvement permette d'obtenir le volume total souhaité de plasma à la fin de la dernière phase de retour.

De nombreuses variantes de l'appareillage précédemment décrit apparaîtront au technicien. A titre d'exemple l'appareillage peut comporter un ballonnet collabable sur la ligne 5 entre la jonction 31 et le clamp 26. Ce ballonnet sert alors de double sécurité avec le capteur de pression 13 en ce sens qu'il se bouche lorsque le débit de la pompe 7 est supérieur à celui de la veine, si le capteur n'a pas fonctionné au cours de la phase de prélèvement. Eventuellement ce ballonnet collabable peut être substitué au capteur 13.

De même le dispositif 8 destiné à transmettre le produit anticoagulant peut éventuellement être omis dans la mesure où l'intérieur de l'aiguille 1, du détecteur de bulles 12 et des lignes 5 et 16 est par exemple recouvert d'un polymère à base de polyétheruréthanes à groupes ammonium héparinés, tels ceux décrits notamment dans le brevet américain 4 046 725. Eventuellement les lignes 5 et 16 peuvent être en un polymère tel que ceux décrits dans le brevet américain cité ci-avant ou en un mélange de polychlorure de vinyle et de polyétheruréthane à groupes ammonium héparinés tel que ceux des mélanges dont il est question dans la demande de brevet européen n° 12 701. La membrane microporeuse peut elle aussi être préparée à partir d'un mélange de polymères selon la demande de brevet européen n° 12 701.

Avec l'appareillage tel que représenté figures 1 et 2 et précédemment décrit, des opérations de plasmaphérèse ont été effectuées sur un donneur en utilisant à titre d'exemple un séparateur 2 à membrane dont la surface membranaire totale est de 600 cm² et comprend deux membranes disposées face à face (constituant le compartiment amont 3) entre lesquelles le sang circule. Les membranes ont chacune 30 cm de longueur et 10 cm de largeur et sont tramées, comme ceci est mieux décrit ci-après. L'épaisseur moyenne du film sang est de 370 microns. Le dispositif 1 de prélèvement est une aiguille de 1,65 mm de diamètre externe et de 1,45 mm de diamètre intérieur. Les canalisations 5 et 6 sont en polychlorure de vinyle (PVC) et ont un diamètre intérieur de

3,5 mm. La canalisation 11 est en PVC et a un diamètre interne de 0,9 mm. La pompe 10 est une pompe péristaltique (marque RP 04 commercialisée par la Société HOSPAL), ladite pompe ayant un corps de pompe en silicone.

Les pompes 7 et 18 sont des pompes péristaltiques (marque RP 01, commercialisées par la Société HOSPAL), lesdites pompes ayant un corps de pompe en silicone. Les récipients 17 et 18 ont une contenance de 1000 cm³ et sont en PVC.

Le capteur 13 est un capteur de marque NATIONAL SEMICON DUCTOR, référence LX 1801 GB, dont la pression affichée est réglée à 1330 Pa (10 mm de mercure) lors de la phase de prélèvement et la valeur maximale de pression est réglée à 13 330 Pa (100 mm de mercure) lors de la phase de retour. Les capteurs 14 et 19 sont des capteurs de même marque et de même référence que le capteur 13. Le capteur 14 est réglé à une pression relative maximale de 10 640 Pa (80 mm de mercure) pour la phase de prélèvement et pour une pression relative minimale de 1330 Pa (10 mm de mercure) lors de la phase de retour, tandis que le capteur 19 est réglé à une pression relative de 1330 Pa (10 mm de mercure) pour la phase de prélèvement et pour une pression relative de 10 640 Pa (80 mm de mercure) pour la phase de retour. Ainsi la pression du sang en circulation est supérieure à la pression du plasma recueilli dans le compartiment 4 aval de l'appareil à membrane qui est à la pression atmosphérique. La pression transmembranaire moyenne est égale à

$$\frac{80+10}{2} = 45 \text{ mm de mercure (5 975 Pa)}.$$

Durant chaque phase de prélèvement le garrot est gonflé à 7980 Pa (60 mm de mercure) et le débit du sang citraté à l'entrée du séparateur est de 85 ml/mm en moyenne.

La membrane utilisée est une membrane tramée obtenue à partir d'une solution dans un solvant organique de polymère que l'on coule sur une trame tournant au contact d'une bande dont la surface est très lisse. Cette solution comprend 8% en poids dans un mélange N-méthylpyrolidone/glycérine (70,8/21,2%) d'un copolymère d'acrylonitrile/méthacrylate de méthyle/méthallyle sulfonate de sodium, comprenant 7,75% en poids de méthacrylate de méthyle et 80 mEg/kg de sites acides. Ce copolymère a une viscosité spécifique de 0,3 à 20 °C en solution à 2 g/l dans le diméthylformamide.

La trame utilisée est un tissu monofilament en polytéréphtalate d'éthylène-glycol, dont la maille est de 75 microns, le diamètre du fil est de 55 microns et le taux de vide est de 33%. Le grammage de cette trame est de 55 g/m².

La membrane tramée microporeuse obtenue a une épaisseur de 120 microns et son grammage est de 10 g de polymère par m² de membrane sèche.

La microstructure de la phase polymère de la membrane est spongieuse et régulière. Sa porosité est de 80%, la porosité étant définie comme le rapport (multiplié par 100) entre le volume des pores sur le volume total de la membrane (polymère + pores).

Le débit à l'eau (additionnée de 1% d'un agent tensio-actif) de cette membrane tramée est de 4,5 ml/h cm$^2$ mmHg.

Le taux de rejet au latex de cette membrane est de:
- 5 à 15% pour un latex calibré à 0,27 microns,
- 65 à 80% pour un latex calibré à 0,4 microns,
- 98 à 100% pour un latex calibré à 0,64 microns.

Avec l'appareillage tel que précédemment défini, en se fixant un volume sang de 350 cm$^3$ lors de la phase de prélèvement, un volume total de 600 cm$^3$ de plasma à recueillir et un rapport volume solution anticoagulant/volume sang de $^1/_{16}$, pendant chaque phase de prélèvement, l'opération de plasmaphérèse a été terminée en 44 minutes après avoir effectué 6 phases de prélèvement et 6 phases de retour.

Le plasma recueilli est pratiquement acellulaire. Il ne contient aucune contamination en globules rouges et seulement 3000 plaquettes par mm$^3$. La concentration protéique du plasma est de 57 g/l.

L'appareillage tel que précédemment décrit peut bien évidemment être utilisé sur les animaux (chien, cheval, ...) notamment pour des opérations de plasmaphérèse.

De façon générale, cet appareillage peut être utilisé chaque fois qu'il est souhaité de ne piquer un sujet (homme ou animal) qu'à un seul endroit. avec une simple aiguille (n'ayant qu'un canal interne), en faisant circuler le liquide prélevé sur le sujet, généralement du sang d'abord dans un sens (phase de prélèvement), puis dans le sens contraire (phase de retour) au contact d'une membrane d'un appareil à membrane, en ayant des moyens pour maîtriser les pressions désirées d'entrée et de sortie du sang de l'appareil à membrane, lors des deux phases. Ainsi avec cet appareillage il est possible d'éliminer des éléments du sang en circulation, une première fois lors de la phase de prélèvement, et une deuxième fois lors de la phase de retour pendant laquelle la fraction du sang n'ayant pas passé à travers la membrane est restituée au sujet. Ainsi l'appareillage tel que précédemment décrit peut être utilisé pour d'autres applications que la plasmaphérèse telle que précédemment définie. En fonction des caractéristiques de séparation (comme par exemple le taux de rejet aux particules calibrées de latex) des membranes utilisées, il sera ainsi par exemple possible de n'appauvrir le sang en circulation que de certaines de ses protéines ou que de certains autres éléments constitutifs du plasma. Il est ainsi également possible avec l'appareillage précédemment décrit de procéder à des séances d'hémofiltration, moyennant par exemple la réinjection d'un liquide de substitution asservie à la quantité de liquide filtré recueilli dans le récipient 20.

Cet appareillage peut être également utilisé pour des opérations d'échange plasmatique, c'est-à-dire en réinjectant à un patient un plasma en quantité équivalente à celle qui lui est soutirée, grâce à une pompe et une canalisation (non représentées) se raccordant par exemple sur la canalisation 5 entre l'entrée 6 du séparateur et le capteur 14, ladite pompe fonctionnant lors de chaque phase de retour. Cet appareillage est utilisable également en dialyse péritonéale, le garrot n'étant plus nécessaire dans ce cas.

Pour répondre aux possibilités d'utilisation définies ci-avant, l'appareillage peut bien entendu comprendre des variantes au niveau de certains de ses éléments structuraux. A titre d'exemple la pompe 18, bien que préférentiellement utilisée, peut éventuellement être remplacée par des moyens équivalents remplissant les mêmes fonctions, c'est-à-dire assurant notamment une certaine pression désirée maximale lors de la phase de retour.

**Revendications**

1. Appareillage utilisable notamment en plasmaphérèse, comprenant:
- un dispositif (1) de prélèvement du sang d'un donneur,
- un appareil (2) à membrane séparant le sang en une fraction ayant traversé la membrane, et en fraction n'ayant pas traversé la membrane,
- une première canalisation (5) reliant le dispositif (1) de prélèvement à l'entrée (6) du compartiment amont (3) de l'appareil (2) à membrane,
- une pompe (7) disposée sur cette canalisation (5),
- un capteur de pression (14) du sang circulant dans la canalisation (5), ledit capteur étant situé entre la pompe (7) et l'entrée (16) du compartiment amont (3),
- un garrot gonflable (21),
- des moyens permettant de gonfler ou de dégonfler le garrot (21),
- une deuxième canalisation (16) reliant la sortie (15) du compartiment amont (3) de l'appareil (2) à membrane à un récipient (17) pour recueillir la fraction du sang n'ayant pas traversé la membrane,
- une pompe (18) disposée sur cette deuxième canalisation (16) et tournant dans le même sens que celui de la pompe (7),
- un capteur de pression (19) disposé sur la canalisation (16) entre ladite pompe (18) et la sortie (15) du compartiment amont (3) de l'appareil à membrane (2),
- un récipient (20) de recueil du plasma ayant traversé la membrane, ce récipient (20) étant en communication avec la sortie du compartiment aval (4) de l'appareil à membrane (2),
- des moyens pour connaître le volume de sang prélevé au donneur lors de la phase de prélèvement, au début de laquelle le garrot (21) est gonflé sur le membre du donneur,
- des moyens pour inverser le sens de rotation des pompes (7 et 18) lorsque la quantité souhaitée de sang est prélevée lors de la phase de prélèvement et assurer ainsi le retour du sang depuis le récipient (17) jusqu'au donneur en passant par les canalisations (16 et 5) et par le compartiment amont (3) de l'appareil à membrane (2), le garrot (21) étant défonflé au début de la phase de retour,

– des moyens (27) permettant la vérification de la fin de la phase de retour et assurant la commutation d'une phase de retour à une phase de prélèvement.

2. Appareillage selon la revendication 1, caractérisé en ce que les moyens permettant de gonfler ou de dégonfler le garrot (21) comprennent une réserve (23) de gaz sous pression, une électrovanne (25) et un manomètre (24) à contact, et en ce que les mouvements de la vanne (25) sont asservis au sens de rotation de la pompe (7).

3. Appareillage selon l'une quelconque des revendications précédentes, caractérisé en ce qu'il comprend un détecteur (12) de bulles et un capteur (13) de pression entre le dispositif de prélèvement (1) et la pompe (7).

4. Appareillage selon l'une quelconque des revendications précédentes, caractérisé en ce qu'il comprend un dispositif (8) pour introduire, lors de la phase de prélèvement, un anticoagulant dans la canalisation (5), à proximité du dispositif (1) de prélèvement.

5. Appareillage selon l'une quelconque des revendications précédentes, caractérisé en ce que le capteur de pression (27) agit sur la pompe (7) et en ce que le capteur (19) de pression agit sur la pompe (18).

6. Appareillage selon l'une quelconque des revendications précédentes, caractérisé en ce que le capteur de pression (14) est réglé pour assurer une pression maximale relative comprise entre 5320 et 13 300 Pa (40 et 100 mm de mercure), du sang entrant dans le séparateur (2) à membrane lors de la phase de prélèvement, tandis qu'il (14) assure une pression relative du sang inférieure à 2660 Pa (20 mm de mercure) lors de la phase de retour, et caractérisé en ce que le capteur de pression (19) permet d'assurer à la sortie (15) de l'appareil (2) à membrane une pression relative du sang inférieure à 2660 Pa (20 mm de mercure) lors de la phase de prélèvement, tandis qu'il permet d'assurer une pression relative maximale du sang, comprise entre 5320 et 13 330 Pa (40 et 100 mm de mercure), lors de la phase de retour, le compartiment (4) aval du séparateur (2) à membrane étant à la pression atmosphérique.

7. Appareillage selon l'une quelconque des revendications 3 à 6, caractérisé en ce que le capteur (13) de pression, agissant sur la pompe (7) permet de s'assurer que la pression du sang dans la portion de la ligne (5) située entre le dispositif de prélèvement (1) et ladite pompe (7) ne descend pas en deçà d'une pression relative minimale prédéterminée, appelée pression de seuil, lors de la phase dite de prélèvement, et en ce que le capteur (13) permet d'assurer que la pression relative ne dépasse pas une valeur maximale lors de la phase de retour.

8. Appareillage selon l'une quelconque des revendications précédentes, caractérisé en ce qu'il comprend de plus des moyens (30) pour connaître à tout moment la quantité de plasma contenue dans le récipient (20).

9. Appareillage selon l'une quelconque des revendications précédentes, caractérisé en ce qu'il comprend une unité logique (22) de commande et de contrôle à laquelle sont reliés notamment les circuits électriques.

10. Appareillage selon l'une quelconque des revendications 8 et 9, caractérisé en ce qu'il comprend de plus un clavier (28) sur lequel on peut choisir le volume de sang que l'on veut faire circuler lors d'une phase de prélèvement et sur lequel (28) on peut choisir le volume total de plasma que l'on veut recueillir, et en ce que l'unité (22) auquel est relié le clavier (28) comprend des moyens pour limiter le volume de circulation du sang lors de la dernière phase de prélèvement et faire en sorte que le volume total de plasma désiré soit obtenu à la fin de la dernière phase de retour.

11. Appareillage selon l'une quelconque des revendications précédentes, caractérisé en ce que la membrane a un taux de rejet au latex, dont les particules sont calibrées à 0,27 microns, inférieur à 75% et en ce qu'elle a un taux de rejet au latex, dont les particules sont calibrées à 0,64 microns, supérieur à 15%.

12. Appareillage selon la revendication 11, caractérisé en ce que la membrane a un taux de rejet au latex, dont les particules sont calibrées à 0,27 microns, inférieur à 30% et en ce qu'elle a un taux de rejet au latex, dont les particules sont calibrées à 0,64 microns, supérieur à 90%.

13. Appareillage selon l'une quelconque des revendications 11 ou 12, caractérisé en ce que la membrane est plane et tramée.

14. Appareillage selon l'une quelconque des revendications précédentes, caractérisé en ce que la membrane a une trame constituée par un tissu monofilament en polytéréphtalate d'éthylène-glycol.

15. Appareillage selon l'une quelconque des revendications 11 ou 12, caractérisé en ce que la trame est constituée d'un tissu dont la maille est de 75 microns et comprend des fils de 55 microns.

16. Appareillage selon l'une quelconque des revendications précédentes, caractérisé en ce que la membrane tramée a une épaisseur comprise entre 50 et 200 microns.

17. Appareillage selon l'une quelconque des revendications précédentes, caractérisé en ce que l'appareil (2) à membrane comprend une membrane à base d'un copolymère d'acrylonitrile, de méthacrylate de méthyle et de méthallylsulfonate de sodium.

18. Appareillage selon la revendication 17, caractérisé en ce que le copolymère comprend 7,75% en poids de méthacrylate et 80 mEg/kg de sites acides.

## Claims

1. Equipment which can be used in particular for plasmapheresis, comprising:

– a device (1) for withdrawing the blood from a donor,

– a membrane apparatus (2) separating the blood into a fraction which has passed through the membrane and into a fraction which has not passed through the membrane,

– a first line (5) connecting the withdrawal device (1) to the inlet (6) of the upstream compartment (3) of the membrane apparatus (2),

– a pump (7) arranged on this line (5),

– a pressure sensor (14) for the blood circulating in the line (5), the said sensor being located between the pump (7) and the inlet (6) of the upstream compartment (3),

– an inflatable tourniquet (21),

– means making it possible to inflate or deflate the tourniquet (21),

– a second line (16) connecting the outlet (15) of the upstream compartment (3) of the membrane apparatus (2) to a vessel (17) for collecting the fraction of the blood which has not passed through the membrane,

– a pump (18) arranged on this second line (16) and rotating in the same direction as that of the pump (7),

– a pressure sensor (19) arranged on the line (16) between the said pump (18) and the outlet (15) of the upstream compartment (3) of the membrane apparatus (2),

– a vessel (20) for collecting the plasma which has passed through the membrane, this vessel (20) being in communication with the outlet of the downstream compartment (4) of the membrane apparatus (2),

– means for measuring the volume of blood withdrawn from the donor during the withdrawal stage, at the start of which the tourniquet (21) is inflatet on the limb of the donor,

– means for reversing the direction of rotation of the pumps (7 and 18) when the desired amount of blood has been withdrawn during the withdrawal stage, and thus ensuring the return of the blood from the vessel (17) to the donor, passing through the lines (16 and 5) and through the upstream compartment (3) of the membrane apparatus (2), the tourniquet (21) being deflated at the start of the return stage, and

– means (27) making it possible to check when the end of the return stage has been reached, and ensuring that a return stage switches to a withdrawal stage.

2. Equipment according to Claim 1, characterized in that the means making it possible to inflate or deflate the tourniquet (21) comprise a reservoir (23) for pressurized gas, an electrovalve (25) and a contact manometer (24), and in that the movements of the valve (25) are controlled by the direction of rotation of the pump (7).

3. Equipment according to either of the preceding claims, characterized in that it comprises a bubble detector (12) and a pressure sensor (13) between the withdrawal device (1) and the pump (7).

4. Equipment according to any one of the preceding claims, characterized in that it comprises a device (8) for introducing an anticoagulant into the line (5), in the vicinity of the withdrawal device (1), during the withdrawal stage.

5. Equipment according to any one of the preceding claims, characterized in that the pressure sensor (27) acts on the pump (7), and in that the pressure sensor (19) acts on the pump (18).

6. Equipment according to any one of the preceding claims, characterized in that the pressure sensor (14) is adjusted so as to ensure a maximum relative pressure of between 5,320 and 13 300 Pa (40 and 100 mm of mercury) of the blood entering the membrane separator (2) during the withdrawal stage, while the said sensor (14) ensures a relative pressure of the blood of less than 2,660 Pa (20 mm of mercury) during the return stage, and characterized in that the pressure sensor (19) makes it possible to ensure, at the outlet (15) of the membrane apparatus (2), a relative pressure of the blood of less than 2,660 Pa (20 mm of mercury) during the withdrawal stage, while it makes it possible to ensure a maximum relative pressure of the blood of between 5,320 and 13 300 Pa (40 and 100 m of mercury) during the return stage, the downstream compartment (4) of the membrane separator (2) being at atmospheric pressure.

7. Equipment according to any one of Claims 3 to 6, characterized in that the pressure sensor (13), acting on the pump (7), makes it possible to ensure that the pressure of the blood in the portion of the line (5) which is located between the withdrawal device (1) and the said pump (7) does not fall below a predetermined minimum relative pressure, called the threshold pressure, during the so-called withdrawal stage, and in that the sensor (13) makes it possible to ensure that the relative pressure does not exceed a maximum value during the return stage.

8. Equipment according to any one of the preceding claims, characterized in that it also comprises means (30) for measuring, at any time, the amount of plasma contained in the vessel (20).

9. Equipment according to any one of the preceding claims, characterized in that it comprises a logic unit (22) for control and monitoring, to which the electrical circuits are connected in particular.

10. Equipment according to either of Claims 8 and 9, characterized in that it also comprises a keyboard (28) on which it is possible to choose the volume of blood which it is desired to circulate during a withdrawal stage, and on which keyboard (28) it is possible to choose the total volume of plasma which it is desired to collect, and in that the unit (22) to which the keyboard (28) is connected comprises means for limiting the volume of blood in circulation during the last withdrawal stage and for ensuring that the total volume of plasma desired is obtained at the end of the last return stage.

11. Equipment according to any one of the preceding claims, characterized in that the membrane has a latex rejection level of less than 75% for particles with a size of 0.27 micron, and in that it has a latex rejection level of more than 15% for particles with a size of 0.64 micron.

12. Equipment according to Claim 11, characterized in that the membrane has a latex rejection level of less than 30% for particles with a size of 0.27 micron, and in that it has a latex rejection level of more than 90% for particles with a size of 0.64 micron.

13. Equipment according to either of Claims 11 and 12, characterized in that the membrane is planar and woven.

14. Equipment according to any one of the preceding claims, characterized in that the membrane has a web consisting of a single-filament fabric made of poly-(ethylene glycol) terephthalate.

15. Equipment according to either of Claims 11 and 12, characterized in that the web consists of a fabric having a mesh size of 75 microns and comprising filaments of 55 microns.

16. Equipment according to any one of the preceding claims, characterized in that the woven membrane has a thickness of between 50 and 200 microns.

17. Equipment according to any one of the preceding claims, characterized in that the membrane apparatus (2) comprises a membrane based on a copolymer of acrylonitrile, methyl methacrylate and sodium methallylsulphonate.

18. Equipment according to Claim 17, characterized in that the copolymer comprises 7.75% by weight of methacrylate and 80 milliequivalents/kg of acid sites.

**Patentansprüche**

1. Anlage, insbesondere für die Plasmapherese, enthaltend
– eine Vorrichtung (1) zur Entnahme von Blut von einem Spender,
– Gerät (2) mit Membran, das das Blut in einen Anteil trennt, der die Membran durchquert hat, und in einen Anteil trennt, der die Membran nicht durchquert hat,
– eine erste Leitung (5), die die Entnahmevorrichtung (1) mit dem Einlass (6) des stromauf gelegenen Abteils (3) des Geräts (2) mit Membran verbindet,
– eine an diese Leitung (5) angeordnete Pumpe (7),
– einen Druckgeber (15) für das in der Leitung (15) zirkulierende Blut, wobei sich der Druckgeber zwischen der Pumpe (7) und dem Einlass (6) des stromauf gelegenen Abteils (3) befindet,
– eine aufblasbare Aderpresse (21),
– Mittel, die das Aufblasen oder Entlüften der Aderpresse (21) gestatten,
– eine zweite Leitung (16), die den Auslass (15) des stromauf gelegenen Abteils des Geräts (2) mit Membran mit einem Gefäss (17) verbindet, das den Anteil des Bluts sammelt, der die Membran nicht durchquert hat,
– eine Pumpe (18), die an dieser zweiten Leitung (16) angeordnet ist und im gleichen Sinn wie demjenigen der Pumpe (7) dreht,
– einen Druckgeber (19), der an der Leitung (16) zwischen der Pumpe (18) und dem Auslass (15) des stromauf gelegenen Abteils (3) des Geräts (2) mit Membran angeordnet ist,
– ein Gefäss (20) zur Aufnahme des Plasmas, das die Membran durchquert hat, wobei dieses Gefäss mit dem Auslass des stromab gelegenen Abteils (4) des Geräts (2) mit Membran verbunden ist,
– Mittel zum Feststellen des Volumens an Blut, das dem Spender während der Entnahmephase entnommen wird, zu deren Beginn die Aderpresse (21) an einem Glied des Spenders aufgeblasen wird,
– Mittel zum Umkehren des Drehsinns der Pumpen (7 und 18), wenn die gewünschte Menge Blut während der Entnahmephase entnommen ist und somit zum Gewährleisten des Rücklaufs des Bluts vom Gefäss (17) zum Spender unter Strömen durch die Leitungen (16 und 5) und durch das stromauf gelegene Abteil (3) des Geräts (2) mit Membran, wobei die Aderpresse (21) zu Beginn der Rücklaufphase entlüftet wird,
– Mittel, die die Kontrolle des Endes der Rücklaufphase gewährleisten und die Umwandlung einer Rücklaufphase in eine Entnahmephase gestatten.

2. Anlage nach Anspruch 1, dadurch gekennzeichnet, dass die Mittel, die das Aufblasen oder Entlüften der Aderpresse (21) gestatten, einen Druckgasvorrat (23), ein Magnetventil (25) und ein Manometer (24) mit Kontakt aufweisen, und dass die Bewegungen des Magnetventils (25) abhängig vom Drehsinn der Pumpe (7) geregelt werden.

3. Anlage nach einem der vorhergehenden Ansprüche, gekennzeichnet durch einen Fühler (12) für Blasen und einen Druckgeber (13) zwischen der Entnahmevorrichtung (1) und der Pumpe (7).

4. Anlage nach einem der vorhergehenden Ansprüche, gekennzeichnet durch eine Vorrichtung (8), die während der Entnahmephase in der Nähe der Entnahmevorrichtung (1) ein Antikoagulationsmittel in die Leitung (5) einführt.

5. Anlage nach einem der vorhergehenden Ansprüche, dadurch gekennzeichnet, dass der Druckgeber (27) auf die Pumpe (7) einwirkt, und dass der Druckgeber (19) auf die Pumpe (18) einwirkt.

6. Anlage nach einem der vorhergehenden Ansprüche, dadurch gekennzeichnet, dass der Druckgeber (14) so eingestellt ist, dass er während der Entnahmephase einen maximalen relativen Druck zwischen 5320 und 13 300 Pa (40 und 100 mm Hg) des in das Trenngerät (2) mit Membran eintretenden Bluts gewährleistet, während er während der Rücklaufphase einen relativen Druck des Bluts von unter 2660 Pa (20 mm Hg) gewährleistet, und dass der Druckgeber (19) am Auslass (15) des Geräts (2) mit Membran während der Entnahmephase einen relativen Druck des Bluts von unter 2660 Pa (20 mm Hg) gewährleistet, während er während der Rücklaufphase einen maximalen relativen Druck des Bluts von zwischen 5320 und 13 330 Pa (40 und 100 mm Hg) gewährleistet, wobei das stromab gelegene Abteil (4) des Trenngeräts (2) mit Membran sich auf atmosphärischem Druck befindet.

7. Anlage nach einem der Ansprüche 3 bis 6, dadurch gekennzeichnet, dass der auf die Pumpe (7) wirkende Geber gewährleistet, dass während der Entnahmephase der Druck des Bluts in dem zwischen der Entnahmevorrichtung und dieser Pumpe (7) gelegenen Teil der Leitung (5) nicht unter einen gegebenen minimalen relativen Druck, genannt Schwelldruck, abfällt, und dass der Geber (13) gewährleistet, dass während der Rücklaufphase der relative Druck einen maximalen Wert nicht übersteigt.

8. Anlage nach einem der vorhergehenden Ansprüche, gekennzeichnet ferner durch Mittel (30) für die Kenntnis der im Gefäss (20) in jedem Augenblick enthaltenden Plasmamenge.

9. Anlage nach einem der vorhergehenden Ansprüche, gekennzeichnet durch eine logische Steuer- und Kontrolleinheit (22), mit der insbesondere die elektrischen Kreise verbunden sind.

10. Anlage nach einem der Ansprüche 8 und 9, dadurch gekennzeichnet, dass sie ferner eine Tasteneinheit (28) enthält, auf der man das Blutvolumen wählen kann, das man während einer Entnahmephase zirkulieren lassen will, und auf der man das gesamte Plasmavolumen wählen kann, das man sammeln will, und dass die Einheit (22), mit der die Tasteneinheit (28) verbunden ist, Mittel enthält zum Begrenzen des Zirkulationsvolumens des Bluts während der letzten Entnahmephase derart, dass das gewünschte gesamte Plasmavolumen am Ende der letzten Rücklaufphase erhalten wird.

11. Anlage nach einem der vorhergehenden Ansprüche, dadurch gekennzeichnet, dass die Membran einen Abweisgrad von unter 75% bei Latex hat, dessen Teilchen auf 0,27 Mikron kalibriert sind, und dass sie einen Abweisgrad von über 15% bei Latex hat, dessen Teilchen auf 0,64 Mikron kalibriert sind.

12. Anlage nach Anspruch 11, dadurch gekennzeichnet, dass die Membran einen Abweisgrad von unter 30% bei Latex hat, dessen Teilchen auf 0,27 Mikron kalibriert sind, und dass sie einen Abweisgrad von über 90% bei Latex hat, dessen Teilchen auf 0,64 Mikron kalibriert sind.

13. Anlage nach einem der Ansprüche 11 oder 12, dadurch gekennzeichnet, dass die Membran eben und eingeschossen ist.

14. Anlage nach einem der vorhergehenden Ansprüche, dadurch gekennzeichnet, dass die Membran einen Schuss hat, der aus einem Monofilamentgewebe aus Poyläthylenglykol-terephthalat besteht.

15. Anlage nach einem der Ansprüche 11 oder 12, dadurch gekennzeichnet, dass der Schuss aus einem Gewebe besteht, dessen Maschenweite 75 Mikron beträgt und das Fäden von 55 Mikron aufweist.

16. Anlage nach einem der vorhergehenden Ansprüche, dadurch gekennzeichnet, dass die eingeschossene Membran eine Dicke von 50 bis 200 Mikron hat.

17. Anlage nach einem der vorhergehenden Ansprüche, dadurch gekennzeichnet, dass das Gerät (2) mit Membran eine Membran auf der Basis eines Acrylnitril-Copolymers, eines Methylmethacrylat-Copolymers und eines Natriummethallylsulfonat-Copolymers aufweist.

18. Anlage nach Anspruch 17, dadurch gekennzeichnet, dass das Copolymer 7,75% Metharylat und 80 mEq/kg saure Stellen aufweist.

figure 1

0 085 016

figure 2

0 085 016